Europäisches Patentamt

European Patent Office

Office européen des brevets

(19)

(11) Numéro de publication: **0 336 865**

**A1**

## (12) DEMANDE DE BREVET EUROPEEN

(21) Numéro de dépôt: **89430007.8**

(22) Date de dépôt: **24.03.89**

(51) Int. Cl.⁴: **A 61 N 1/05**
**A 61 F 11/04**

(30) Priorité: **08.04.88 FR 8804854**

(43) Date de publication de la demande:
**11.10.89 Bulletin 89/41**

(84) Etats contractants désignés:
**AT BE CH DE ES FR GB GR IT LI LU SE**

(71) Demandeur: **MXM, Société Anonyme**
**"Les Mimosas" Quartier Croix-Rouge**
**F-06600 Antibes (FR)**

(72) Inventeur: **Charvin, Guy**
**25, Chemin de la Peyrigoue Parc Saint Honoré**
**F-06600 - Antibes (FR)**

(74) Mandataire: **Azais, Henri et al**
**c/o CABINET BEAU DE LOMENIE 14, rue Raphael**
**F-13008 Marseille (FR)**

(54) **Dispositifs porte-électrodes implantables dans la cochlée pour stimuler électriquement le nerf auditif.**

(57) L'invention a pour objet des dispositifs porte-électrodes implantables dans la cochlée pour stimuler électriquement le nerf auditif.

Un porte-électrodes selon l'invention comporte un corps (6), en un matériau flexible et biocompatible. par exemple en silicone. Il comporte des électrodes (11) en un métal conducteur, par exemple en platine, dont une partie au moins est apparente et épouse le contour du corps (6) et dont une partie est noyée dans celui-ci. Chaque électrode est connectée à un conducteur noyé dans le corps (6) qui relie l'électrode à une tête réceptrice implantée sous la peAU. Le corps (6) est divisé en tronçons successifs par des fentes (12) qui s'étendent sur une partie de la section transversale et qui créent des charnières d'articulation, de sorte que le porte-électrodes épous facilement la courbure de la cochlée et soit autos-orientable pour conserver une orientation choisie préférentielle desdites électrodes, conforme à la configuration de la cochlée.

Une application est la correction de la surdité totale.

Fig.8

EP 0 336 865 A1

## Description

### Dispositifs porte-électrodes implantables dans la cochlée pour stimuler électriquement le nerf auditif.

La présente invention a pour objet des dispositifs porte-électrodes implantables dans la cochlée, notamment dans la cochlée d'un être humain, pour stimuler le nerf auditif par des signaux électriques délivrés par un appareil électronique de prothèse auditive.

Le secteur technique de l'invention est celui de la construction des prothèses auditives électroniques.

On connaît des équipements ou appareils électroniques de prothèse auditive destinés à corriger les surdités profondes qui comportent des électrodes implantées dan la cochlée qui sont destinées à transmettre des signaux électriques aux fibres du nerf auditif qui se trouvent situées dans la cochlée.

On rappelle brièvement que les ondes acoustiques sont d'abord transmises par le tympan et par les osselets aux liquides de l'oreille interne qui excitent, à leur tour, les cellules sensorielles ciliées de la cochlée ou limaçon.

Chez les êtres vivants atteints de surdité profonde, les cellules sensorielles ciliées sont déficientes et les prothèses auditives classiques, qui ont pour effet d'accroître l'énergie acoustique, sont sans effet.

Il est connu, depuis une vingtaine d'années, que l'on peut corriger les surdités profondes au moyen de prothèses auditives dites oreilles bioniques, qui comportent, d'une part, un appareil externe équipé d'un microphone qui capte les sons et de circuits électroniques qui décomposent les sons en signaux électriques de différentes fréquences et, d'autre part, d'électrodes qui sont connectées auxdits circuits électroniques soit directement, soit par induction à travers la peau et qui sont placées sur un porte-électrodes qui est implanté dan la cochlée au cours d'une intervention chirurgicale.

La demande de brevet FR. A. 2.383.657 (BERTIN et Cie et Al) décrit un équipement électronique de prothèse auditive comportant un émetteur externe qui est couplé par induction à un implant récepteur comportant un porte-électrodes intracochléaire.

La présente invention a pour objet un dispositif porte-électrodes qui peut être utilisé avec un émetteur externe du type décrit dans ce brevet antérieur ou avec tout autre émetteur externe analogue.

La demande de brevet européen EP. A1. 183.605 (BERTIN et Cie) décrit des dispositifs porte-électrodes destinés à être implantés dans la cochlée, qui comportent un support en matériau isolant biocompatible, dans lequel sont creusées des cavités qui débouchent toutes sur une même face du support et qui contiennent chacune une électrode.

La demande de brevet EP A3 0.007.157 (UNIVERSITE DE MELBOURNE), décrit des porte-électrodes destinés à être implantés dans la cochlée humaine, comportant un tube flexible, autour duquel sont enroulées des électrodes régulièrement espacées constituées, par exemple, de feuilles de platine.

Une autre demande de brevet EP A2 0.215.726 sous priorité d'une demande australienne de cette même Université et dont l'inventeur est Monsieur CLARK G. décrit un porte-électrodes comportant au moins, près de son extrémité, une discontinuité constituée, soit par une réduction de la section, soit par un changement de matériau, affectant dans les deux cas toute la section du porte-électrode, de façon à faciliter la déflexion de l'extrémité quand elle rencontre un obstacle lors de son implantation.

La demande de brevet EP. A. 002.226 (Carl HANSEN) décrit des porte-électrodes destinés à être implantés dans la cochlée qui comportent un support en un matériau isolant ayant la forme d'une fourche ayant deux branches dont chacune a une longueur qui correspond à celle d'une spire de la cochlée, dans laquelle cette branche est introduite et chaque branche porte plusieurs électrodes qui sont situées d'un même côté de la branche.

La demande de brevet EP. A. 002.068 (Carl HANSEN) décrit des porte-électrodes destinés à être implantés dans la cochlée, qui comportent un support allongé et flexible, ayant la forme d'une feuille, lequel support est précourbé avec une courbure longitudinale temporaire qui correspond sensiblement à la courbure de l'axe du canal de la cochlée et est libéré après avoir été mis en place dans la cochlée pour revenir à son état de courbure propre qui permet d'obtenir un engagement optimum entre les terminaisons nerveuses et les électrodes portées par le support, lesquelles électrodes sont situées le long de l'un des bords du support.

On rappelle que la cochlée a la forme générale d'un tube conique, ayant une longueur d'environ 35 mm qui est logé dans un canal osseux en forme de colimaçon comportant deux spires et demi. En section transversale, le tube est divisé en trois canaux qui sont remplis de liquide. Ces canaux sont désignés respectivement : rampe tympanique, rampe vestibulaire et canal cochléaire. Le canal cochléaire est séparé de la rampe tympanique par la membrane basilaire sur laquelle se trouvent les cellules sensorielles ciliées. Les porte-électrodes sont placés généralement dans la rampe tympanique.

Le chirurgien engage l'extrémité du porte-électrodes à travers la fenêtre située à l'extrémité de la rampe tympanique et il pousse le porte-électrodes pour le faire avancer à l'intérieur de la rampe, ce qui exige que le porte-électrodes soit suffisamment rigide pour transmettre la poussée et suffisamment souple pour suivre un conduit hélicoïdal pendant au moins deux tours. En effet, il est nécessaire que le porte-électrodes s'étende sur la plus grande longueur de la cochlée car la cochlée comporte dans le sens de la longueur des zones successives qui sont sélectivement excitées par certaines fréquences, ce qui permet au cerveau de reconnaître les fréquences sonores.

Les résultats obtenus avec des porte-électrodes tels que ceux qui sont décrits dans les document précédemment cités, ne sont pas toujours satisfaisants, notamment au point de vue de l'intelligibilité des sons perçus par le patient.

L'insertion d'un porte-électrodes en spirale à l'intérieur de la cochlée en poussant sur celui-ci peut conduire à ce que le porte-électrodes se comporte comme un burin qui détériore les membranes et les terminaisons des nerfs auditifs. Ce danger est signalé dans les trois brevets antérieurs EP. A2 0.215.726, EP. A. 002.226 et EP.A. 002.068, qui cherchent à y remédier, soit en créant une discontinuité ponctuelle à l'extrémité, soit en divisant le porte-électrodes en deux branches qui sont insérées chacune dans une seule spire de la cochlée, soit en conférant au porte électrodes, une précourbure temporaire, ce qui conduit à des solutions complexes.

Par ailleurs, des essais réalisés sur un modèle de cochlée avec des porte-électrodes conformes à ceux qui sont décrits dans le document EP. A1. 183.605 et ceci serait le cas également avec des porte-électrodes conformément au brevet EP. A2. 0.215.726, ont montré que, lorsqu'on insère ce porte-électrodes dans une cochlée, il tend inévitablement à se vriller pour suivre la courbure longitudinale de la cochlée, de sorte que les électrodes sont orientées dans toutes les directions au lieu d'être dirigées vers la membrane basilaire. Il en résulte que les lignes de courant issues de certaines électrodes ne sont pas captées ou sont captées très faiblement par certaines zones sensibles de la cochlée d'où une distorsion des sons qui rend ceux-ci difficilement intelligibles.

Les porte-électrodes décrits dans le brevet EP. 0.007.157 qui utilisent des électrodes circulaires ne présentent pas cet inconvénient. Cependant, les électrodes circulaires créent des lignes de courant qui divergent dans toutes les directions et il en résulte que l'énergie émise par chaque électrode se disperse et seule une petite partie atteint les terminaisons nerveuses.

Le problème que l'invention se propose de résoudre est de procurer des porte-électrodes destinés à être insérés dans la rampe tympanique d'une cochlée par la fenêtre d'extrémité de celle-ci qui améliorent l'intelligibilité des sons et ne blessent pas la paroi de la cochlée.

A cet effet, il faut obtenir des porte-électrodes qui doivent être suffisamment rigides pour pouvoir être poussés pour les forcer à progresser dans les spires de la rampe tympanique et assez flexibles pour épouser la forme hélicoïdale sans détériorer les parois de la rampe ou les terminaisons nerveuses et qui doivent de plus, ne pas se tordre autour de leur axe longitudinal, de sorte que les électrodes situées tout au long du porte-électrodes soient placées face aux terminaisons nerveuses et le plus près possible de celles-ci.

La solution au problème posé est constituée par des porte-électrodes implantables dans la cochlée pour stimuler électriquement le nerf auditif, du type comportant un support flexible, dont le corps est une tige de forme allongée en un matériau biocompatible incluant au moins une discontinuité mécanique près de son extrémité, lequel support flexible porte des électrodes reliées chacune par un conducteur à des moyens de transmission des signaux électriques, délivrés par un appareil électronique externe, qui capte les sons et qui les transforme en signaux électriques, caractérisé en ce qu'une partie au moins de l'extrémité dudit corps du support flexible qui porte des électrodes est composée de tronçons reliés entre eux par lesdites discontinuités mécaniques, et celles-ci s'étendent sur une partie seulement de la section transversale et sont disposées dissymétriquement par rapport à l'axe dudit support flexible, de telle façon que deux tronçons successifs restent reliés entre eux par des points de contacts permanents, sont articulés en charnière autour de cesdits points en pouvant pivoter l'un par rapport à l'autre, de sorte qu'ils rendent le dispositif auto-orientable en conservant pendant leur insertion dans la cochlée leur orientation choisie conformément à la configuration de celle-ci.

Selon un mode de réalisation préférentiel, discontinuités mécaniques sont des fentes transversales qui s'étendent sur une partie de la tige, de telle façon que la partie non fendue de celle-ci est continue et constitue lesdites charnières entre deux tronçons.

Avantageusement, ces fentes sont situées dans chaque intervalle entre deux électrodes successives.

Avantageusement, chaque électrode comporte une partie apparente qui épouse la forme de la surface du corps du support et les parties apparentes de toutes les électrodes sont placées d'un même côté du support qui est le côté situé à l'intérieur de la cochlée lorsque le porte-électrodes est inséré dans celle-ci, c'est-à-dire en regardant vers la pointe, le côté gauche pour un porte-électrodes destiné à une oreille droite et le côté droit pour un porte-électrodes destiné à une oreille gauche.

Selon un mode de réalisation préférentiel, chaque électrode comporte une tête dont une partie arrondie est apparente et épouse une partie du contour du support flexible et une queue qui est noyée dans la masse du support. De préférence, la queue est coudée en forme de crosse.

L'invention a pour résultat de nouveaux porte-électrodes destinés à être insérés dans la cochlée d'un être vivant, notamment dans une cochlée humaine, pour stimuler le nerf auditif au moyen de signaux électriques reçus d'un appareil portatif externe qui capte les sons, qui les convertit en signaux électriques et qui filtre ceux-ci pour les décomposer en bandes de fréquence déterminées qui sont envoyées chacune sur une des électrodes.

Grâce à la division en tronçons ou segments articulés les uns aux autres par des charnières constituées par exemple par des fentes transversales, un porte-électrodes selon l'invention a une raideur suffisante pour pouvoir être poussé vers l'avant et une flexibilité telle qu'il suit facilement la courbure de la cochlée sur un tout et demi sans risquer de blesser la membrane qui délimite la rampe tympanique dans laquelle il est inséré et sans se vriller, c'est-à-dire sans tourner autour de son axe longitudinal, de sorte qu'il est possible de placer la partie apparente de toutes les électrodes du même côté du porte-électrodes et d'être certain qu'une fois le porte-électrodes mis en place dans la cochlée, toutes les parties apparentes des électrodes, qui sont au contact du liquide contenu dans la rampe tympanique, se trouvent orientées vers l'intérieur de la cochlée, c'est-à-dire vers les cellules sensitives du nerf auditif.

Il en résulte qu'un porte-électrodes selon l'invention permet d'obtenir une bonne stimulation des cellules nerveuses à partir des signaux électriques et améliore l'intelligibilité de sons par le patient, ce qui est le résultat recherché.

La description suivante se réfère aux dessins annexés qui représentent, sans aucun caractère limitatif, des exemples de réalisation de porte-électrodes selon l'invention.

La figure 1 est une coupe axiale d'un porte-électrodes selon l'invention inséré dans une cochlée humaine.

La figure 2 est une vue d'ensemble de la partie interne d'une prothèse auditive comportant un porte-électrodes selon l'invention.

Les figures 3 à 5 sont des vues de dessus de plusieurs modes de réalisation de porte-électrodes destinés à une oreille droite.

Les figures 6 à 8 sont respectivement, une vue de dessus, une vue en élévation et une vue en perspective d'un porte-électrodes destiné à une oreille droite.

Les figures 9A, 9B et 9C sont des vues de dessus et en perspective d'autres exemples de réalisation de porte-électrodes destinés à des oreilles droites.

La figure 10 est une coupe selon XX de la figure 8.

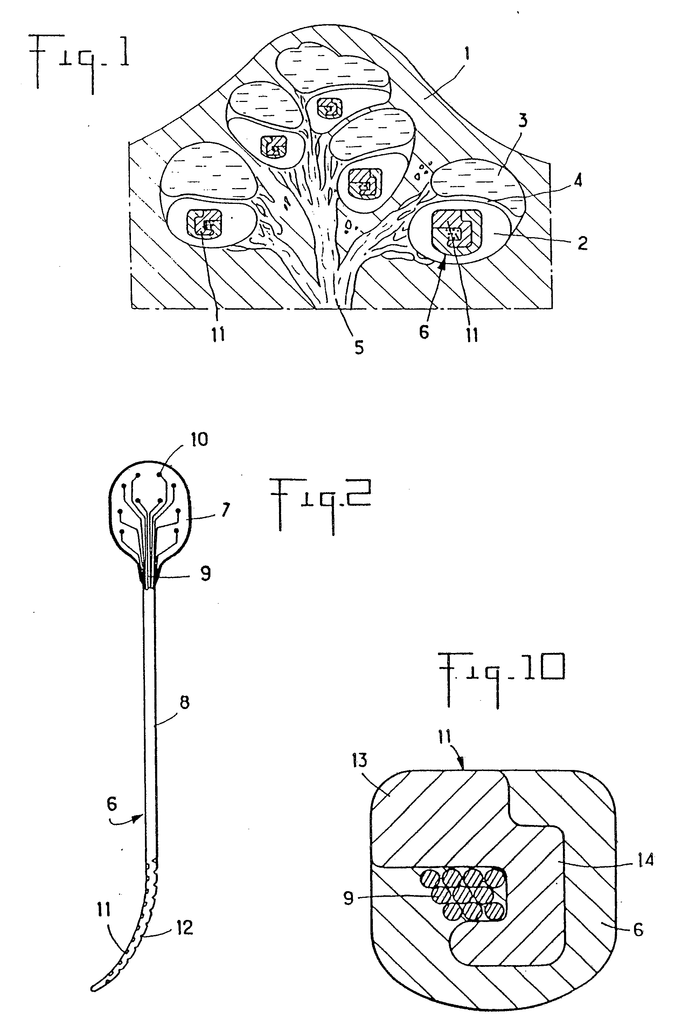

La figure 1 est une coupe axiale d'une cochlée humaine. Celle-ci présente la forme d'un conduit hélicoïdal ou en spirale creusé à l'intérieur de l'os temporal 1 et enroulé autour d'un axe osseux conique appelé columelle. Ce canal fait environ deux tours et demi autour de la columelle. Il contient deux conduits délimités par des membranes et remplis de lymphe la rampe tympanique 2 et la rampe vestibulaire 3, qui sont séparées entre elles par une membrane 4 dite membrane basilaire. Le nerf auditif 5 comporte un tronc commun situé dans l'axe et des ramifications dont les terminaisons se trouvent sur la membrane basilaire. Ces ramifications nerveuses comportent des cellules sensorielles ciliées, qui constituent l'organe de corti, qui captent normalement les vibrations acoustiques du liquide contenu dans les deux rampes et qui convertissent celles-ci en influx nerveux qui sont transmis au cerveau.

Les cellules sensorielles ciliées réparties le long de la cochlée capteur sélectivement les fréquences sonores.

On a représenté sur la figure 1 un porte-électrodes 6 inséré dans la rampe tympanique 2.

Après avoir pratiqué une ouverture dans l'os temporal 1, le chirurgien introduit l'extrémité distale du porte-électrodes dans la fenêtre ronde située à l'extrémité de la rampe tympanique, puis il pousse le porte-électrodes qui doit progresser vers le sommet de la cochlée en tournant autour de la columelle.

Le porte-électrodes 6 est engagé dans la cochlée sur une longueur d'environ 20 mm et celle-ci est garnie d'électrodes réparties sur toutes la longueur, par exemple douze électrodes.

Si l'on regarde une cochlée de la base vers le sommet, la cochlée de l'oreille droite tourne dans le sens trigonométrique tandis que la cochlée de l'oreille gauche tourne dans le sens des aiguilles d'une montre.

La figure 2 représente une vue d'ensemble d'un dispositif porte-électrodes selon l'invention qui est implanté à l'intérieur de l'os temporal. Celui-ci comporte une tête réceptrice 7, qui est placée sous la peau, derrière l'oreille, en regard d'une tête émettrice qui est disposée à l'extérieur et qui émet des signaux électriques provenant d'un appareil portatif qui est porté par le patient et qui comporte un microphone. Les signaux émis par la tête émettrice sont transmis par induction à la tête réceptrice qui ne comporte donc aucune source d'énergie.

Le dispositif récepteur selon la figure 2 comporte un corps allongé ou support 8 en un matériau isolant biocompatible, par exemple en silicone. A l'intérieur de ce corps passent des petits filaments conducteurs 9 qui sont isolés les uns des autres. Chaque filament a une extrémité 10 située dans la tête réceptrice et il relie celle-ci à une électrode de stimulation 11 portée par l'extrémité distale de la tige 8.

Les électrodes 11 sont réparties sur une longueur de tige qui est de l'ordre de 2 cm à partir de l'extrémité distale, cette longueur correspondant à une partie de la largeur d'une cochlée.

On rappelle que les cochlées de tous les individus ont sensiblement la même taille et que la cochlée d'un individu est formée dès la première enfance et ne change pas de dimensions au cours de l'existence, de sorte qu'une seule taille de porte-électrodes peut être utilisée pendant toute l'existence d'un individu.

On voit sur la figure 2 que toutes les électrodes 11 sont disposées d'un même côté du porte-électrodes.

On voit sur la figure 1 que les ramifications du nerf auditif aboutissant à la membrane basilaire se situent du côté intérieur de la cochlée vers le tronc commun 5, que ces ramifications vont rejoindre.

Pour obtenir la meilleure transmission possible des signaux électriques entre les électrodes et les terminaisons nerveuses, il est donc préférable que les électrodes se situent du côté des spires qui se trouvera placé à l'intérieur. Dans le cas d'un porte-électrodes destiné à une oreille droite dont la cochlée tourne dans le sens trigonométrique, les électrodes 11 sont placées du côté gauche du porte-électrodes en regardant vers l'extrémité distale de celui-ci.

Inversement, dans le cas d'un porte-électrodes destiné à être placé dans une oreille gauche qui tourne dans le sens des aiguilles d'une montre, les électrodes 11 sont placées du côté droit du porte-électrodes en regardant vers l'extrémité distale de celui-ci.

Le problème à résoudre est de fabriquer des porte-électrodes qui conservent leur orientation pendant leur insertion dans la cochlée, de sorte que les électrodes de stimulation 11 restent toutes placées du côté intérieur sur toute la longueur du porte-électrodes.

En effet, si on insère dans un circuit en colimaçon une tige flexible ayant par exemple une section circulaire, les fibres internes se trouvent comprimées et les fibres externes mises en tension et ces contraintes internes entraînent des mouvements de torsion de la tige autour de son axe.

Le problème posé est résolu au moyen de porte-électrodes dans lesquels la partie distale qui porte les électrodes et qui est engagée dans la cochlée est constituée de tronçons qui sont reliés entre eux par des charnières autour de chacune desquelles deux tronçons successifs peuvent pivoter l'un par rapport à l'autre.

Un porte-électrodes découpé en tronçons successifs articulés présente l'avantage de transmettre les poussées, de sorte qu'il peut être facilement inséré dan la cochlée et en même temps, de se courber facilement pour épouser la forme hélicoïdale de la cochlée sans se tordre autour de son axe.

La figure 2 représente un mode de réalisation dans lequel la partie distale comporte des fentes transversales 12 qui s'étendent sur une partie de la section transversale du support et qui sont situées du côté opposé aux électrodes 11, c'est-à-dire du côté externe lorsque le porte-électrodes est courbé.

La figure 3 représente à plus grande échelle, une vue de dessus de l'extrémité distale d'un porte-électrodes destiné à être inséré dans une oreille droite.

Le corps 6 en matériau biocompatible porte des électrodes 11 en métal inerte biocompatibles, par exemple en platine.

Les électrodes 11 sont localisées sur le côté gauche en regardant vers la pointe. Le corps 6 est divisé en tronçons successifs par des fentes 12 qui sont situées du côté opposé à celui qui porte les électrodes 11 et entre deux électrodes.

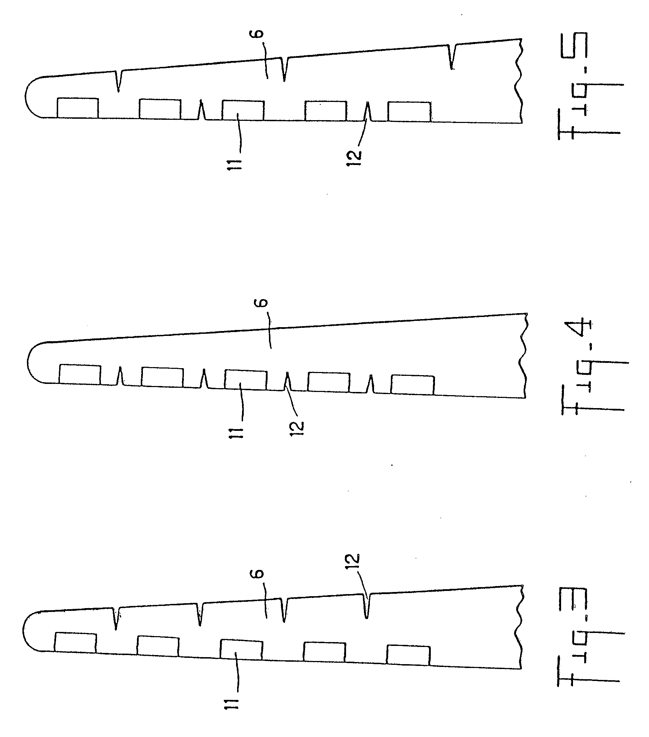

La figure 4 représente une vue de dessus d'un autre mode de réalisation d'un porte-électrodes destiné à être inséré dans une oreille droite, dans lequel le corps 6 porte des électrodes 11 et des fentes 12 intercalées entre les électrodes, du même côté que celles-ci.

La figure 5 représente une vue de dessus d'un autre mode de réalisation d'un porte électrodes destiné à être inséré dans une oreille droite, dans lequel le corps 6 porte des électrodes 11 situées du côté gauche en regardant vers la pointe et porte des fentes 12 situées des deux côtés.

Les porte-électrodes destinés à être insérés dans l'oreille gauche sont symétriques, c'est-à-dire que les électrodes 11 sont situées du côté droit en regardant vers la pointe.

Lorsqu'on insère un porte-électrodes selon l'une quelconque des figures 3 à 5 dans une cochlée d'oreille droite, le corps 6 fléchit et s'enroule dans le sens trigonométrique et les fentes 12 facilitent la flexion et évitent que des contraintes de flexion et/ou de compression ne prennent naissance dan les figures externes et/ou internes, ce qui a pour effet d'éviter que le corps 6 ne se vrille en pivotant autour de son axe longitudinal, de sorte que les électrodes 11 restent disposées à l'intérieur de la courbe, c'est-à-dire du côté des terminaisons du nerf auditif et à proximité de celles-ci.

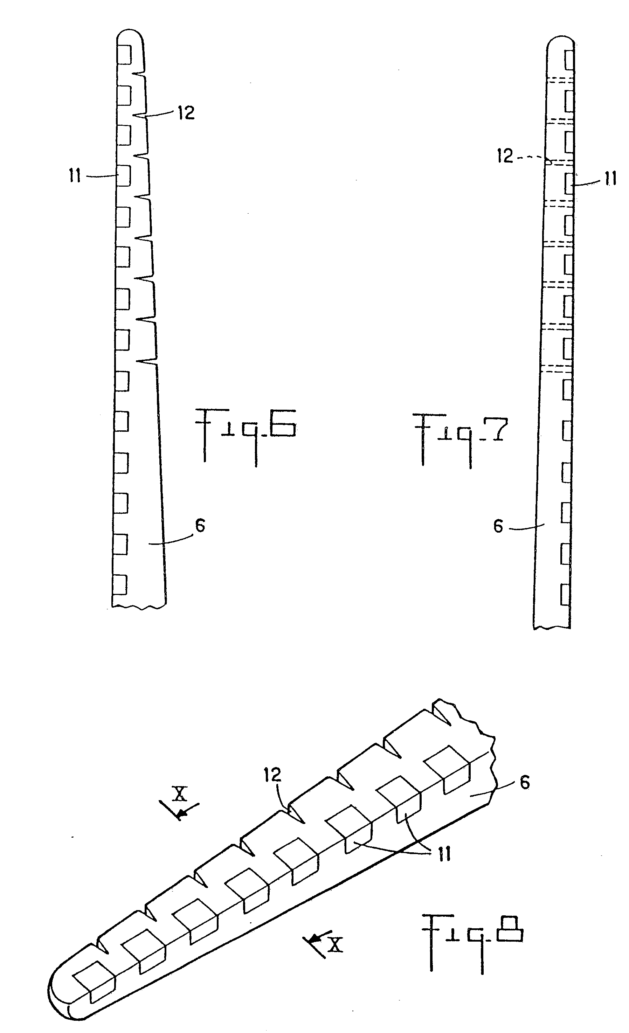

Les figures 6 et 7 représentent respectivement une vue de dessus et une vue en élévation de l'extrémité distale d'un porte-électrodes destiné à être inséré dans la cochlée d'une oreille droite. Le corps 6 est en silicone. Il porte quinze électrodes 11 réparties le long du côté gauche en regardant vers la pointe. La partie distale est divisée en tronçons par huit fentes 12, situées du côté opposé aux électrodes et au milieu des huit intervalles situés entre les neuf électrodes d'extrémité. Par contre, la partie arrière où se situent les six dernières électrodes, qui occupe l'entrée rectiligne de la cochlée, n'est pas divisée en tronçons.

La figure 8 est une vue en perspective de l'extrémité distale d'un porte-électrodes dont le corps 6 présente une section transversale ayant une forme générale hémisphérique à angles arrondis.

Les électrodes 11 sont placées à cheval sur l'arête située du côté supérieur gauche en regardant vers la pointe. Les fentes 12 coupent la face latérale droite et sont situées au milieu de chaque intervalle entre deux électrodes.

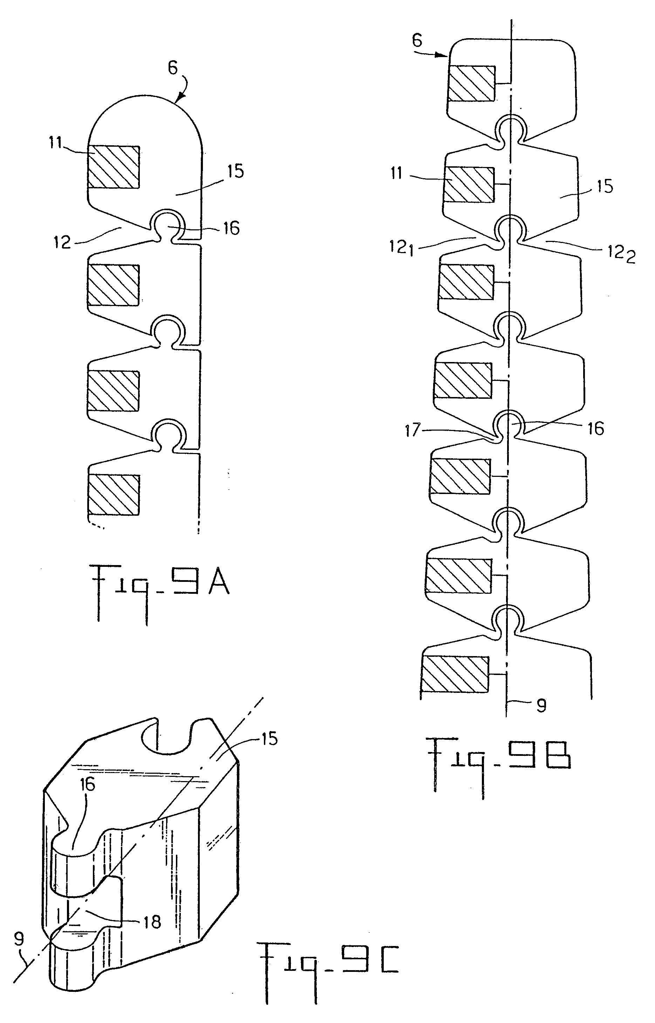

Les figures 9A et 9B sont des vues de dessus de deux autres exemples de réalisation de porte-électrodes suivant l'invention, toujours destinées à une oreille droite. Les tronçons 15 portant les électrodes 11 sont ici indépendants les uns des autres et sont reliés entre eux par de véritables charnières 16 assurant leur articulation les uns par rapport aux autres dans un plan déterminé perpendiculaire à l'axe de ces charnières et dans un sens préférentiel, soit par exemple grâce à un décalage de leur axe par rapport à celui du corps 6 comme dans la figure 9A, soit grâce à un profil dissymétrique des fentes 12 comme dans la figure 9B, constituant ainsi une structure de type chaîne de vélo à courbure préférentielle.

Dans la figure 9A, les fentes 12 sont situées d'un seul côté pour permettre le rapprochement des tronçons 15 par pivotement vers ce même côté, et de l'autre côté les éléments tronçons viennent même en appui entre eux quand le corps 6 est présenté droit.

Dans la figure 9B, les fentes 12 sont disposées de part et d'autre des charnières 16 mais avec un profil 17 dissymétrique des tronçons autour de celles-ci.

Ces charnières 16 sont constituées par exemple d'une partie mâle cylindrique d'un côté, s'encastrant dans une partie femelle correspondante de l'autre.

Les fils 9 reliant les électrodes 11 passent alors d'un tronçon 15 au suivant, de plusieurs manières possibles, telles que par exemple : soit ils sont disposés à côté des charnières et passent en arrière au travers desdites fentes $12_2$, avec une légère surlongueur permettant le mouvement d'extension de celles- ci; soit ils passent au travers des charnières 16 elles-même dont les parties mâles sont alors évidées pour permettre, grâce à la souplesse des fils et de la matière comme représenté sur les figures 9B et 9C leur rotation, soit ils sont interrompus au niveau desdites charnières qui comportent alors des contacts tournants face à face, sur chaque partie mâle et femelle assurant alors la continuité électrique et quel que soit l'angle de rotation.

La figure 9C est une vue perspective schématique d'un tronçon 15 sans son électrode et tel que représenté dans les figures 9A et 9B précédentes, avec un passage de fils 9 dans le plan de l'axe d'articulation de la charnière 16. La partie mâle de celle-ci est évidée en son milieu pour constituer la zone 18 de passage des fils qui autorisent alors par leur souplesse l'auto-orientation du porte-électrodes.

La figure 10 est une coupe transversale selon XX d'un porte-électrodes selon la figure 8.

On voit sur cette coupe une électrode 11 qui comporte une tête 13 et une queue 14.

Une partie du contour de la tête 13 qui est apparent épouse une partie du contour de la face supérieure et de la face latérale gauche du corps 6. La queue 14 est noyée dans la masse du corps 6. Elle présente une forme de crosse coudée et les conducteurs 9 qui relient électriquement les électrodes à la tête réceptrice 7 sont logés dans la crosse. L'un des conducteurs est soudé à chacune des électrodes.

Lorsqu'un porte-électrodes selon les figures 8 et 10 est inséré dan une cochlée, la face supérieure plane est placée parallèlement à la membrane basilaire et la partie apparente des électrodes se trouve placée du côté intérieur de la cochlée à proximité immédiate des terminaisons du nerf auditif, de sorte que les signaux émis par les électrodes stimulent efficacement les cellules nerveuses.

Pour fabriquer un porte-électrodes selon l'invention, on place les électrodes 13 et les conducteurs 9 dans un moule dans lequel on coule ensuite de la silicone. Les fentes transversales 12 peuvent être obtenues par des arêtes internes du moule ou bien en sectionnant transversalement le corps après l'avoir démoulé.

Dans toutes les réalisations suivant l'invention tels que dans les exemples décrits, le corps 6 du porte-électrodes est divisé en tronçons successifs séparés par des discontinuités telles que des fentes ouvertes 12. Il est précisé que ces discontinuités telles que les fentes et telles que les charnières comme décrit dans les figures 9, peuvent être bouchées et recouvertes d'un matériau élastomère biocompatible suffisamment déformable et différent de celui qui compose le corps 6, de sorte que l'on conserve ainsi les discontinuités des propriétés mécaniques telles que décrites précédemment, et telles qu'elles permettent d'assurer les propriétés d'auto-orientation du porte-électrodes pendant son insertion dans la cochlée, tout en ayant un porte-électrodes qui se courbe facilement et présente une surface externe uniforme.

La présente invention n'est pas limitée aux modes de réalisations décrits ci-dessus et qui ne constituent que des exemples de fabrication de dispositifs porte-électrodes suivant l'invention et des modifications et des variantes peuvent être apportées dans le cadre de celle-ci.

## Revendications

1. Dispositif porte-électrodes implantable dans la cochlée pour stimuler électriquement le nerf auditif, du type comportant un support flexible (6) dont le corps est une ligne de forme allongée en un matériau biocompatible incluant au moins une discontinuité mécanique près de son extrémité, lequel support flexible porte des électrodes (11) reliées chacune par un conducteur (9) à des moyens (10) de transmission des signaux électriques, délivrés par un appareil électronique externe, qui capte les sons et qui les transforme en signaux électriques, caractérisé en ce qu'une partie au moins de l'extrémité dudit corps du support flexible (6) qui porte des électrodes est composée de tronçons reliés entre eux par lesdites discontinuités mécaniques (12), et celles-ci s'étendent sur une partie seulement de la section transversale et sont disposées dissymétriquement par rapport à l'axe dudit support flexible, de telle façon que deux tronçons successifs restent reliés entre eux par des points de contacts permanents, sont articulés en charnière autour de cesdits points en pouvant pivoter l'un par rapport à l'autre, de sorte qu'ils rendent le dispositif auto-orientable en conservant pendant leur insertion dans la cochlée leur orientation choisie conformément à la configuration de celle-ci.

2. Porte-électrodes selon la revendication 1, caractérisé en ce que lesdites discontinuités mécaniques (12) sont des fentes transversales qui s'étendent sur une partie de la tige (6), de telle façon que la partie non fendue de celle-ci est continue et constitue lesdites charnières entre deux tronçons.

3. Porte-électrodes suivant la revendication 1, caractérisé en ce que les tronçons (15) portant les électrodes (11) sont indépendants les uns des autres et reliés entre eux par de véritables charnières (16) constituant lesdits points de contact permanents et assurant leur articulation les uns par rapport aux autres, dans un plan déterminé perpendiculaire à l'axe de cesdites charnières et dans le sens choisi conformément à la configuration de ladite cochlée, lesquelles charnières (16) peuvent être constituées d'une partie mâle cylindrique d'un côté, s'encastrant dans une partie femelle correspondante de l'autre.

4. Porte-électrodes selon l'une quelconque des revendications 1 à 3, caractérisé en ce que lesdites électrodes (11) comportent des parties apparentes à la surface dudit corps qui sont placées d'un même côté dudit corps (6) qui est le côté situé à l'intérieur de la cochlée lorsque le porte-électrodes est inséré dans celle-ci.

5. Porte-électrodes selon la revendication 4, destiné à être inséré dans la cochlée d'une oreille droite, caractérisé en ce que les parties apparentes desdites électrodes (11) sont situées du côté gauche en regardant vers la pointe.

6. Porte-électrodes selon la revendication 4, destiné à être inséré dans la cochlée d'une oreille gauche, caractérisé en ce que les parties apparentes desdites électrodes (11) sont situées du côté droit en regardant vers la pointe.

7. Porte-électrodes selon l'une quelconque des revendications 4 à 6, caractérisé en ce que ledit corps (6) est divisé en tronçons successifs par des fentes (12) qui s'étendent sur une partie de la section transversale dudit corps et qui sont situées dans chaque intervalle entre deux électrodes successives.

8. Porte-électrodes selon l'une quelconque des revendications 1 à 7, caractérisé en ce que lesdites électrodes (11) comportant une tête (13) dont une partie arrondie est apparente et épouse une partie du contour externe dudit corps (6) et une queue (14) qui est noyée dans la masse dudit support (6).

9. Porte-électrodes selon la revendication 8, caractérisée en ce que ladite queue (14) est coudée en forme de crosse et lesdits conducteurs (9) sont logés dans l'espace délimité par ladite tête (13) et par ladite crosse (14).

10. Porte-électrodes selon l'une quelconque des revendications 1 à 9, caractérisé en ce que lesdites discontinuités mécaniques, telles les fentes (12) et les charnières (16) sont bouchées et recouvertes d'un matériau élastomère biocompatible suffisamment déformable, de sorte que ledit porte-électrode présente une surface externe uniforme et, assure et conserve les propriétés d'auto-orientation données par lesdites discontinuités mécaniques dissymétriques.

Fig.1

Fig.2

Fig.10

Fig. 3

Fig. 4

Fig. 5

Fig.6

Fig.7

Fig.8

6

11 15

12 16

Fig. 9A

6

11 15

121 122

17 16

9

Fig. 9B

15

16

18

9

Fig. 9C

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A,D | EP-A-0 215 726 (CLARK) <br> * En entier * <br> --- | 1,2,4, 10 | A 61 N 1/05 <br> A 61 F 11/04 |
| A | FR-A-2 403 084 (CEA) <br> * En entier * <br> ----- | 5,6,8 | |

DOMAINES TECHNIQUES RECHERCHES (Int. Cl.4)

A 61 N
A 61 B

Le présent rapport a été établi pour toutes les revendications

| Lieu de la recherche | Date d'achèvement de la recherche | Examinateur |
|---|---|---|
| LA HAYE | 22-06-1989 | LEMERCIER D.L.L. |